# EUROPEAN PATENT APPLICATION

(11) **EP 4 079 752 A1**
(43) Date of publication of application: **26.10.2022**
(21) Application number: 20897565.6
(22) Date of filing: 10.11.2020
(51) Int. Cl.: C07K 14/42, C07K 16/40, C12Q 1/37, G01N 33/50, G01N 33/531, G01N 33/543, G01N 33/574

(54) **STANDARD SUBSTANCE FOR PSA QUANTIFICATION, PREPARATION METHOD THEREFOR, STANDARD SOLUTION FOR PSA QUANTIFICATION, AND PSA QUANTIFICATION METHOD**

(30) Priority: 06.12.2019 JP 2019221264
(71) Applicant: Otsuka Pharmaceutical Co., Ltd., Tokyo 101-8535 (JP)
(72) Inventor: KANEKO, Tomonori, Tokyo 100-7015 (JP); KAYA, Takatoshi, Tokyo 100-7015 (JP); OTANI, Makiko, Tokyo 100-7015 (JP)
(74) Representative: Hoffmann Eitle
(86) International application number: PCT/JP2020/041874
(87) International publication number: WO 2021/111820

(57) **Abstract**

An object of the present invention is to provide a standard substance for quantification of PSA having a specific sugar chain that can be used in a general purpose quantification, wherein the standard substance has less unbalanced sugar chain expression patterns, can be manufactured with high reproducibility, and enables the quantification of patient's sample comprising a high concentration of PSA, and preparation method therefor, standard solution for PSA quantification, and PSA quantification method. The standard substance comprises a compound having the structure of a PSA with a sugar chain represented by any of the following formulae A to D, and is isolated and purified from a natural product, chemically or enzymatically altered from a natural product, or the compound is artificially synthesized.

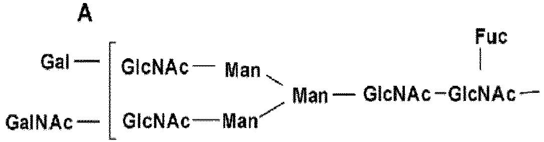

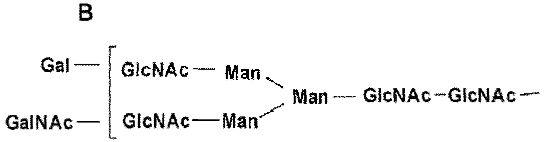

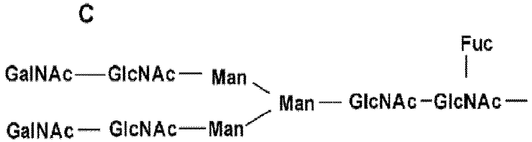

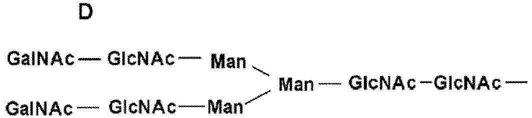

## Description

### Technical field

The present invention relates to a standard substance for PSA quantification, a preparation method therefor, a standard solution for PSA quantification, and a PSA quantification method. The present invention relates in more detail to a standard substance for quantification of PSA with a specific sugar chain, The present invention relates in more detail to a standard substance for quantification of PSA with a specific sugar chain, which has less unbalanced sugar chain expression patterns, which can be manufactured with high reproducibility, and which enables general-purpose quantification including the quantification of patient's sample comprising a high concentration of PSA.

### Background art

It is known that a prostate specific antigen (hereinafter called "PSA"), which indicates the development of prostatic diseases such as prostatic cancer or prostatic hypertrophy, is a protein secreted from glandular cells of the prostate gland into glandular cavities of the prostate gland, and is a protein expressed specifically in the prostate gland. PSA is not a protein expressed specifically in prostatic cancer, and increases also due to diseases other than prostatic cancer such as prostatic hypertrophy and prostatitis.

PSA is a glycoprotein having one asparagine-binding sugar chain (also called a "N-glycan chain"). It is known that PSAs of prostatic cancer patients have altered terminal sugar chain structures in its N-glycan chain, or have a highly branched complex sugar chain. It is considered that the PSAs of prostatic cancer patients have specific sugar chains.

It is known that, for example, the amount of PSA having an N-acetyl-D-galactosamine-β-(1,4)-N-acetyl-D-glucosamine (hereinafter called "LacdiNAc") residue (hereinafter called "LacdiNAc-PSA") in blood samples derived from prostatic cancer patients is higher than that in blood samples derived from healthy persons or patients with benign diseases such as prostatic hypertrophy.

It is observed that the sugar chain profile of PSA is altered due to the onset of prostatic cancer, the PSA with a specific sugar chain increases, and the PSA with the specific sugar chain is at a high concentration in blood samples of prostatic cancer patients. In the above, the "specific sugar chain" has an N-acetyl-D-galactosamine-β-(1,4)-N-acetyl-D-glucosamine residue at an end. Said specific sugar chains have been confirmed to have high affinities for WFA lectin. This type of PSAs having the altered sugar chain as above are collectively called as PSA-glycosylation isomer (PSA-Gi).

In contrast, because no such alteration of sugar chain as above occurs upon the development of prostatic hypertrophy, no change in the concentration of the PSA with the specific sugar chain in prostatic hypertrophy patients is observed.

It is, therefore, known that prostatic cancer patients and prostatic hypertrophy patients can be distinguished by measuring the concentration of PSA with the specific sugar chain in blood samples.

The inventors of the present application have reported that prostatic cancer and benign diseases such as prostatic hypertrophy can be distinguished by measuring the amount of a component attached to the PSA molecule having a high affinity for WFA lectin (for example, refer to Patent Literature 1) with superior performance over the existing PSA inspections.

A standard substance comprising PSA with a structure and expression profile of sugar chain similar to those of the specific sugar chain secreted in prostatic cancer patient samples that have been subjected to cancerous alteration needs to be provided to quantify the prostate specific antigen-glycosylation isomer (PSA-Gi) contained in the samples of a patient suspected of developing prostatic cancer with high accuracy and reproducibility.

Samples containing PSAs subjected to cancerous alteration having the specific sugar chains (PSA-Gi) at high concentrations might be used as conventional standard substances. However, different patients or donors may have different sugar chain structure sequences, and significantly different reactivities against, for example, antibodies or lectin. Conventional standard substances therefore have problems with respect to, for example, the continuity and reproducibility of values.

There has been a method for quantifying the PSA having the specific sugar chain by fluorescence spectroscopy. The concentration of the PSA having the specific sugar chain in a sample was quantified with reference to the measurement result (i.e. signal) of serially diluted PSA produced or secreted by cultured cells contained in the culture supernatant. For example, refer to Patent Literature 1. There has also been a method in which the PSA with the specific sugar chain in a sample was quantified with reference to serially diluted pooled cancer patient's serums containing the PSA at high concentrations.

When PSA produced by cultured cells or when PSA in pooled cancer patients' serum is used as the "standard PSA", the quantification of the PSA having the specific sugar chain has the following disadvantages.
(1) Since only a very small portion of secreted PSA proteins in a patient's serum or a cell culture solution have the specific sugar chain, for example, around 1 to 5% in a patient sample, the amount of the produced PSAs having the specific sugar chain is very small, the dynamic range of the calibration curve plotted in the relationship between the concentration and the signal is narrow, the quantifiable concentration range is limited, and it is difficult to quantify a sample containing a high concentration of the target PSA.
(2) When serum derived from a patient or the antigen derived from specific cultured cells is used as it is, it is assumed that sugar chain structure patterns are unbalanced. Good recognition performance expected when a standard substance having a sugar chain structure close to various patient samples is used may not be obtained, the versatility is low, and false positives or false negatives may be produced.
(3) The modification patterns of sugar chains are very complicated, and 30 or more structures are known as sugar chain modification types on PSA molecules. When a PSA derived from a patient or PSA derived from specific cultured cells is used as a standard substance as it is, it is therefore very difficult to stably and continuously provide PSAs that exhibit the consistent properties at the time of change in the lot of raw materials for the standard substance.

A standard substance which can be used in a general-purpose quantification that enables the quantification of patient's samples comprising high concentration of PSA, and which has less unbalanced sugar chain expression patterns has been desired for the above-mentioned reasons. It is also necessary that the standard substance can be supplied stably and continuously.

### Citation List

### Patent Literature

Patent Literature 1:
Japanese Patent Laid-Open No. 2013-076666

### Summary of Invention

### Technical Problem

The present invention has been completed in view of the above-mentioned problems and situations, and objects thereof to be achieved include to provide a standard substance for quantification of PSA with a specific sugar chain, which has less unbalanced sugar chain expression patterns, which can be manufactured with high reproducibility, and which enables general-purpose quantification including the quantification of patient's sample comprising a high concentration of PSA.

Objects of the invention also include to provide a preparation method of the standard substance, a standard solution for PSA quantification, and a PSA quantification method.

### Solution to Problem

The present inventors have found that PSA having a specific residue at a non-reducing terminal of the sugar chain has an important relationship with specific cancers in the process of examining PSA sugar chain profiles related to the onset of cancers such as prostatic cancer to solve the above-mentioned problems and completed the present invention.

Thus, the above-mentioned problems related to the present invention are solved by the following means.

A standard substance for quantification of prostate specific antigen (hereinafter referred to as "PSA") having a specific sugar chain,
wherein the standard substance comprises a compound having the structure of a PSA with a sugar chain represented by any of the following formulae A to D: 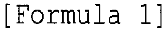 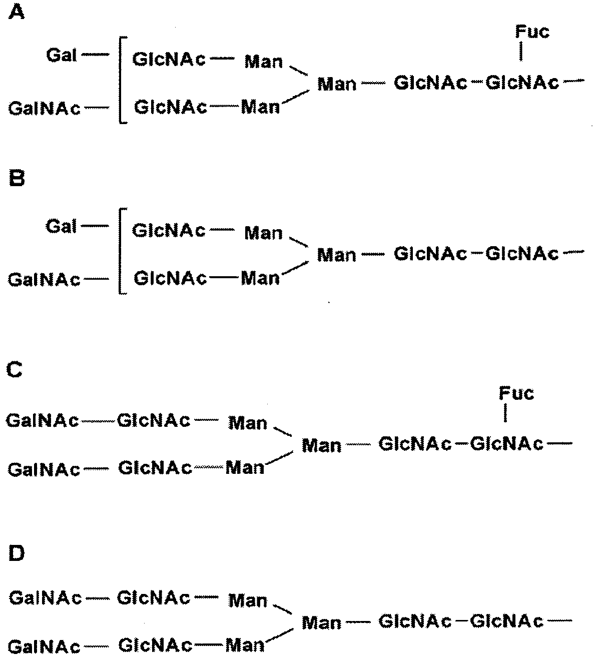 wherein the compound is isolated and purified from a natural product, the compound is chemically or enzymatically altered from a natural product, or the compound is artificially synthesized.

2. A method for preparing the standard substance for PSA quantification of item 1, which is any method of the following (1) to (4):
(1) a method which comprises isolating, purifying, diluting or concentrating a PSA with a sugar chain of interest from PSAs in a raw material that is derived from a human, cultured cells, or fungi,
(2) a method which comprises chemically or enzymatically decomposing or synthesizing a sugar chain on a PSA in a raw material that is derived from a human, cultured cells, or fungi to form PSA with a sugar chain of interest, and isolating, purifying, diluting, or concentrating the PSA with the sugar chain of interest,
(3) a method which comprises exchanging the sugar chain on a PSA in a raw material that is derived from a human, cultured cells or fungi with an artificially synthesized sugar chain by utilizing a chemical or enzymatic substitution reaction, and
(4) a method which comprises manipulating a gene encoding for an enzyme involved in the sugar chain biosynthesis in cells or fungi by means of gene recombination technology, and using the cells or fungi to produce the compound.

3. A standard solution for PSA quantification, comprising the standard substance for PSA quantification according to item 1, wherein the standard solution comprises a PSA with a sugar chain represented by any of formulae A to D.

4. The standard solution for PSA quantification according to item 3, comprising the PSA having the sugar chains of formula A or B, wherein the total amount of both the PSAs having the sugar chain of formula A or B is 80% by mass or more of the total amount of the PSAs having the sugar chain of any one of formulae A to D.

5. The standard solution for PSA quantification according to item 3, comprising 30 to 70% by mass of PSA with the sugar chain of formula A, 20 to 50% by mass of PSA with the sugar chain of formula B, 1 to 10% by mass of PSA with the sugar chain of formula C, and 1 to 10% by mass of PSA with the sugar chain of formula D on the basis of the total amount of PSAs having a sugar chain of any of formulae A to D.

6. A method for quantifying PSA using the standard substance for PSA quantification according to item 1 or the standard solution for PSA quantification according to any one of item 3 to item5, comprising:
a measurement step in which a signal derived from PSA to be quantified in a sample is measured; and
a quantification step in which converting the signal derived from the PSA of interest measured in the measurement step into a quantitative result to give amount of the PSA of interest based on the relationship between the signal derived from the PSA of interest and the amount of the PSA of interest,
wherein, in the quantification step, at least one PSA with a sugar chain represented by any of formulae A to D is used as a reference sample, concentrations of the reference sample in the standard substance are adjusted to predetermined concentrations, the signal from the reference sample is obtained by using the same method as in the measurement step, and the relationship between the obtained signal and the predetermined concentrations is used as the quantitative information.

7. The method for quantifying PSA according to item 6, wherein the measurement procedure used in the measurement step is column chromatography, mass spectrometry, or ligand binding assay.

8. The method for quantifying PSA according to item 7, wherein surface plasmon field-enhanced fluorescence spectroscopy is used for the detection in the ligand binding assay.

9. The method for quantifying PSA according to any one of item 6 to item 8, wherein a lectin and an antibody whose affinities for PSA having β-N-acetylgalactosamine residue at a terminal of the sugar chain are higher than that for PSA not having β-N-acetylgalactosamine residue at any terminal of the sugar chain are used as capture molecules.

10. The method for quantifying PSA according to item 9, wherein the lectin and the antibody are labeled with a fluorescence dye.

11. The method for quantifying PSA according to item 9 or item 10, wherein the lectin is *Wisteria floribunda* agglutinin (WFA), soybean agglutinin (SBA), *Vicia villosa* lectin (VVL), or *Trichosanthes japonica* agglutinin (TJA-II).

12. The method for quantifying PSA according to any one of item 7 to item 11, wherein the lectin and the antibody are used as secondary capture molecules for the ligand binding assay.

### Advantageous Effects of Invention

According to the present invention, a standard substance for quantifying PSA with a specific sugar chain, which has less unbalanced sugar chain expression patterns, which can be manufactured with high reproducibility, and which enables general-purpose quantification including the quantification of patient's sample comprising a high concentration of PSA is provided. In this invention, a preparation method therefor, a standard solution for PSA quantification, and a PSA quantification method are also provided.

The mechanism of expression or action of PSAs having the specific sugar chain relating to the present invention remains unclear.

A general purpose quantification including quantification of PSAs with the specific sugar chain in samples containing high concentration of PSA become feasible by measuring the amount of PSA with the specific sugar chain in a test sample based on a calibration curve obtained by using the standard substance for PSA quantification of the present invention. The standard substance can be provided with high production reproducibility and less unbalanced sugar chain expression patterns.

The cultured cells are obtained by immortalizing cells that are extracted from highly malignant tissues or metastatic sites in a patient. Sugar chains that are expressed in the early stages of prostatic cancer or before prostate cancer acquires metastatic potential share some common structures. It is suggested that the sugar chain structures expressed in the cultured cells becomes more complicated, and the number of cancer-specific sugar chain residues at the sugar chain terminal increases.

It has been confirmed that when the sugar chains recognized by a molecule such as WFA lectin increase in density in a sample, a polyvalent effect is generally produced, and the binding amount of the molecule increases. A sample including many PSAs that present sugar chains of interest therefore have greatly different reactivity per sugar chain molecule from a sample wherein not many PSAs have the sugar chains of interest. It is inferred that the standard substance which is a patient sample or a secretion from a prostatic cancer cell line with high malignancy and high PSA concentration is a standard substance having an unbalanced reactivity of the sugar chain recognition molecule such as the WFA lectin.

Since information on, for example, the cancer development, cancer onset and cancer malignancy of a subject related to the test sample can be precisely generated by using the standard substance of the present invention, the cancer development, cancer onset, or cancer malignancy of the subject can be rapidly evaluated by simple and noninvasive methods, and the burdens of the patients and doctors can be reduced.

### Brief Description of Drawings

[Figure 1] Figure 1 is a conceptual diagram schematically showing reaction steps in measurement with a SPFS measuring device.
[Figure 2] Figure 2 is a figure showing examples of calibration curves prepared using various solutions containing PSA.
[Figure 3] Figure 3 is a figure showing an example of the results of an experiment for confirming the purities of standard samples by electrophoresis.
[Figure 4] Figure 4 is a figure showing an extra ion chromatogram of the important sugar chain structure components.
[Figure 5] Figure 5 provides the structures of PSA sugar chains represented by formulae A to F.
[Figure 6] Figure 6 provides the contents of the sugar chains in different production lots of the standard substance.
[Figure 7A] Figure 7A is a table showing dilution linearity test results.
[Figure 7B] Figure 7B is a figure showing dilution linearity test results.
[Figure 8] Figure 8 is a schematic diagram showing the outline of a SPFS measuring device.
[Figure 9] Figure 9 is a partially enlarged view of the SPFS measuring device of Figure 8.

### Description of Embodiments

The standard sample for prostate specific antigen (hereinafter referred to as "PSA") quantification of the present invention is a standard substance for the quantitative analysis of PSAs having a specific sugar chain, and is characterized in that the standard sample comprises a compound having a structure of PSA having a sugar chain represented by any of the formulae A to D, wherein the compound is isolated and purified from a natural product, the compound is chemically or enzymatically modified from a natural product, or the compound is artificially synthesized.

This characteristic is a technical characteristic common or corresponding to the following embodiments.

The method for preparing a standard substance for PSA quantification of the present invention is a method for preparing the standard substance for PSA quantification, and is characterized by being any preparation method of the above discussed (1) to (4).

The standard solution for PSA quantification of the present invention is a standard solution for PSA quantification comprising the standard substance for PSA quantification, and is characterized by comprising PSA with a sugar chain represented by any of the formulae A to D. The standard solution of the present invention enables general-purpose quantification including the quantification of patient's sample comprising a high concentration of PSA. The standard substance can be provided with high production reproducibility and have less unbalanced sugar chain expression patterns.

It is preferable from the viewpoint of solving the problem related to the present invention that the standard solution comprises a PSA having a sugar chain of formula A or B, wherein the total amount of both the PSAs having the sugar chain of formula A or B is 80% by mass or more of the total amount of the PSAs having the sugar chain of any of formulae A to D in the sample. Further, it is still more preferable that the standard solution comprises 30 to 70% by mass of a PSA with the sugar chain of formula A, 20 to 50% by mass of a PSA with the sugar chain of formula B, 1 to 10% by mass of a PSA with the sugar chain of formula C, and 1 to 10% by mass of a PSA with the sugar chain of formula D on the basis of the total amount of the PSAs with a sugar chain of any one of formulae A to D.

A PSA quantification method of the present invention is a method for quantifying PSA using the standard substance for PSA quantification or the standard solution for PSA quantification, comprises:
a measurement step in which a signal derived from PSA to be quantified in a sample is measured; and
a quantification step in which converting the signal derived from the PSA of interest measured in the measurement step into a quantitative result to give amount of the PSA of interest based on the relationship between the signal derived from the PSA of interest and the amount of the PSA of interest,
wherein, in the quantification step, at least one PSA with a sugar chain represented by any of formulae A to D is used as a reference sample, concentrations of the reference sample in the standard substance are adjusted to predetermined concentrations, the signal from the reference sample is obtained by using the same method as in the measurement step, and the relationship between the obtained signal and the predetermined concentrations is used as the quantitative information.
General-purpose quantification that enables the quantification of patient's samples comprising a high concentration of PSA with respect to PSAs having the specific sugar chain also with the sugar chain expression patterns less unbalanced is enabled using the PSA quantification method.

In one embodiment, it is preferable from the viewpoints of accuracy, simplicity, and the like that the measurement method used in the measurement step is column chromatography, mass spectrometry, or ligand binding assay. It is preferable to use surface plasmon field-enhanced fluorescence spectroscopy for the detection of signals in the ligand binding assay.

In the present invention, it is preferable to use a lectin and an antibody whose affinities for PSA having β-N-acetylgalactosamine residue at a terminal of the sugar chain are higher than those for PSAs not having β-N-acetylgalactosamine residue at any terminal of the sugar chain as capture molecules.

It is also preferable that the lectin and the antibody are labeled with a fluorescence dye. Furthermore, it is preferable that the lectin is *Wisteria floribunda* agglutinin (WFA), soybean agglutinin (SBA), a *Vicia villosa* lectin (VVL), or *Trichosanthes japonica* agglutinin (TJA-II). It is preferable from the viewpoints of accuracy to use the lectin and the antibody as secondary capture molecules for the ligand binding assay.

Hereinafter, the present invention and its features, and modes and embodiments for implementing the present invention will be described in detail. In the present application, "-" or "to" between numeral values is used as an expression that means the numerical values are included as the lower limit and the upper limit.

In the present application, the "natural product" refers to a material produced by organisms, namely living things such as animals, plants, and microorganisms.

An "analyte" (also referred to as "sugar chain to be detected", an "object to be detected", or a "test substance") is a specific sugar chain on a glycoprotein (antigen) to be used as a biomarker or a PSA having a specific sugar chain, and refers to a sugar chain intended to be bound to the lectin and the antibody for detection to detect the analyte or PSA with said sugar chain.

The meanings of the other terms used herein will be suitably described.

### 1. Standard substance for PSA quantification

The standard substance for prostate specific antigen (hereinafter referred to as "PSA") quantification of the present invention is used for the quantitative analysis of PSAs having a specific sugar chain, and is characterized in that the standard substance comprises a compound having the structure of PSA with a sugar chain represented by any of the following formulae A to D, wherein the compound is isolated and purified from a natural product, the compound is chemically or enzymatically modified from a natural product, or the compound is artificially synthesized.

Here, the "PSA with a specific sugar chain" refers to a PSA having the target sugar chain among various glycosylation isomers that have various sugar chains in a sample. "Glycosylation isomer" refers collectively to PSAs having a sugar chain in which various sugars are sequenced in various orders, that is, PSAs modified with a sugar chain having a structure in which various sugars are sequenced in various orders, are collectively referred to as PSA-glycosylation isomer (abbreviated as "PSA-Gi").

The "standard substance for PSA quantification" refers to a compound suitable for establishing a standard that enables the quantitative measurement of the PSA in a sample.

### 1.1 Sugar chain structure of the standard substance

The standard substance for PSA quantification of the present invention comprises a PSA having a sugar chain resented by any of the following formulae A to D.

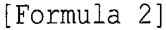

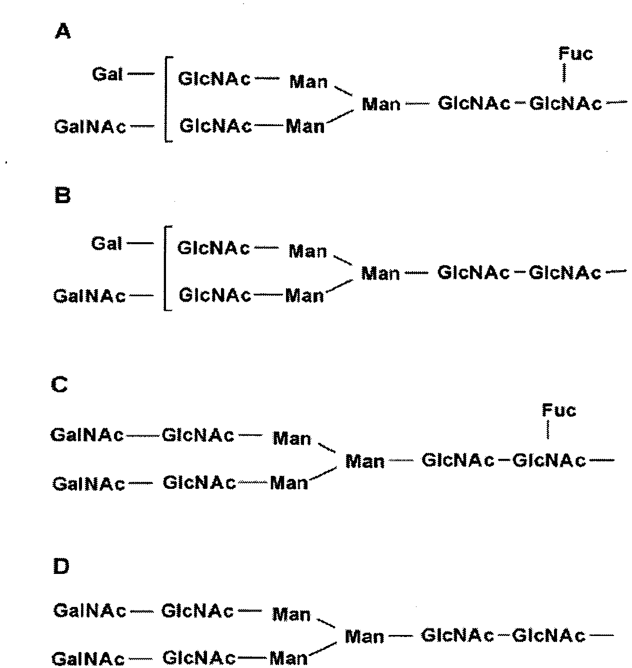

The abbreviated signs of sugar residues in the above-mentioned formulae mean as follows:
Gal: galactose
GalNAc: N-acetylgalactosamine
GlcNAc: N-acetylglucosamine
Man: mannose
Fuc: fucose

By using the PSAs with a sugar chain represented by the above formula as standard substance (reference compound), the present invention can provide standard substance for quantification of PSA with a specific sugar chain, which has less unbalanced sugar chain expression patterns, which can be manufactured with high reproducibility and which enables general-purpose quantification including the quantification of patient's sample comprising a high concentration of PSA.

When a standard solution containing the standard substance having the above-mentioned structure is prepared, the standard substance can be used singly or in combinations of two or more depending on the object. The PSA-modifying sugar chain sequences in a plurality of prostatic cancer cell lines and a plurality of prostatic cancer patient's serums were analyzed by mass spectrometry and the like. As a result, the sugar chain structures corresponding to the formulae A to D were detected, and it has been presumed that the sugar chains of formulae A to D are the main components among components having terminal LacdiNAc structures.

### 1.2 Method for preparing standard substance for PSA quantification

The standard substance for PSA quantification of the present invention may be prepared by any of the following methods. A plurality of the following methods may be employed in combination.
(1) a method which comprises isolating, purifying, diluting or concentrating a PSA with a sugar chain of interest from PSAs in a raw material that is derived from a human, cultured cells, or fungi,
(2) a method which comprises chemically or enzymatically decomposing or synthesizing a sugar chain on a PSA in a raw material that is derived from a human, cultured cells, or fungi to form PSA with a sugar chain of interest, and isolating, purifying, diluting, or concentrating the PSA with the sugar chain of interest,
(3) a method which comprises exchanging the sugar chain on a PSA in a raw material that is derived from a human, cultured cells or fungi with an artificially synthesized sugar chain by utilizing a chemical or enzymatic substitution reaction, and
(4) a method which comprises manipulating a gene encoding for an enzyme involved in the sugar chain biosynthesis in cells or fungi by means of gene recombination technology, and using the cells or fungi to produce the compound.

For example, according to the above-mentioned preparation method (1), human seminal plasma or cell culture supernatant may be subjected to an anti-PSA antibody column chromatography to purify the substance by affinity to the antibody. Then, PSA with a specific sugar chain having the ability to bind to WFA lectin is isolated and purified using a WFA lectin column chromatography. Culture supernatant of the cultured cells expressing a sugar chain having a high affinity for WFA lectin is alternatively concentrated to produce the standard substance.

As the above-mentioned preparation method (2), for example, sialic acid at the terminal of a sugar chain is hydrolyzed using a glycohydrolase, neuraminidase to alter the sugar chain on a PSA derived from seminal plasma into a PSA-modifying sugar chain that is specifically secreted from cancer cells. Then, only PSA modified with a sugar chain having an affinity for the WFA lectin or the like (molecules that exhibit reactivity with the LacdiNAc structure containing N-acetyl galactosamine, for example, a lectin, a sugar chain-recognizing antibody, an aptamer, or the like) is isolated, purified, and concentrated from seminal plasma-derived PSA subjected to hydrolysis treatment by affinity column chromatography.

As the above-mentioned preparation method (3), for example, seminal plasma-derived PSA is treated with ENGase (endo-β-N-acetylglucosaminidase), which is an enzyme that eliminates the N-type sugar chain of a glycoprotein, and the sugar chain on the PSA is substituted with an artificially synthesized N-type sugar chain using the modified hydrolase endo CC N180H, and is isolated and purified.

As the above-mentioned preparation method (4), for example, a clone in which the expression of the sugar chain sequence of interest is enhanced may be obtained by altering glycosyltransferase of, for example, CHO cells and HEK293 cells by a gene recombinant technique to produce PSA with the sugar chain of interest, and the PSA is isolated and purified.

The purity of the PSA (PSA-Gi) isolated and purified as protein can be measured, for example, by electrophoresis. The sequence of the sugar chains on the isolated and purified PSAs can be comprehensively analyzed by using LC-MS and the content of the specific sugar chain of the PSAs can be calculated. The standard substance can therefore be prepared based on these measurement and analysis results so that the standard substance contains the sugar chains in a desired ratio.

### 2. Standard solution for PSA quantification

The standard solution for PSA quantification of the present invention comprises the standard substance for PSA quantification, and is characterized by comprising PSA with a sugar chain represented by any of the formulae A to D.

### 2.1 Composition of standard solution for PSA quantification

It is preferable from the viewpoints of quantification accuracy and versatility that the standard solution comprise PSAs having the sugar chain represented by formula A or B, and the total amount of both the PSAs is 80% by mass or more of the total amount of the PSAs having a sugar chain represented by formulae A to D.

It is preferable from the viewpoints of still higher quantification accuracy and versatility that the standard solution comprises 30 to 70% by mass of the PSA with the sugar chain of formula A, 20 to 50% by mass of the PSA with the sugar chain of formula B, 1 to 10% by mass of the PSA with the sugar chain of formula C, and 1 to 10% by mass of the PSA with the sugar chain of formula D on the basis of the total amount of the PSAs having any one of formulae A to D sugar chains.

Mass spectrometric analysis had revealed that many samples are presumed to contain PSAs having A-D sugar chains in the above ratio and therefore, the above-mentioned composition is effective for a general-purpose standard substance.

### 2.2 Method for preparing standard solution for PSA quantification

As the method for preparing the standard solution for PSA quantification, the standard substance for PSA quantification may be incorporated into a suitable solvent in a predetermined amount is preferable.

Examples of the solvent for the standard solution include buffer solutions. The buffer solutions may be any of those commonly used in this field and are not particularly limited. Examples may include buffer solutions having a buffering action to maintain the pH usually in the range of pH 5.0 to 10.0, and preferably around neutral, i.e. pH 6.5 to 8.5. Specific examples include tris-HCl buffer solution, tris buffered saline solution, MES buffer solution, HEPES buffer solution, borate buffer solution, phosphate buffer solution, phosphate buffered saline, veronal buffer, and good's buffer. The concentrations of the buffering agent in these buffer solutions may suitably be selected from the range of 5 to 1000 mM, and preferably 5 to 300 mM.

The buffer solution may further contain substances such as a sensitizer, a surfactant, an antiseptic (for example, sodium azide, salicylic acid, and benzoic acid), a stabilizer (for example, albumin, globulin, water-soluble gelatin, a surfactant, and a saccharide), an activator, and others that are used in this field and do not inhibit the stability with coexistent reagents or do not inhibit antigen-antibody reaction. The concentration ranges of these reagents may be suitably selected from concentration ranges commonly used in well-known measurement methods.

### 2.3 Preparation of calibration curve

The PSA quantification method of the present invention is characterized by using the relationship between the signal obtained by measuring standard solutions containing a reference compound which is a PSA with a sugar chain represented by at least one of formulae A to D and the concentrations of the reference compound in the solution. The concentrations of the reference compound in the standard solutions are varied and adjusted to a plurality of predetermined values.

In one embodiment, it is preferable to prepare a calibration curve beforehand based on the relationship between the concentrations of the PSA, that is the reference compound and the intensities of the signals derived from the reference compound. The PSA concentration corresponding to the signal intensity derived from the PSA to be quantified in a sample can be subjected to determination, namely quantification, based on the relationship between the PSA concentrations and the signal intensities shown in this calibration curve.

### 3. Method for quantifying PSA using the standard solution for quantification

The method for quantifying PSA using the standard solution or standard substance for quantification of the present invention, comprises:
(a) a measurement step in which a signal derived from PSA to be quantified in a sample is measured; and
(b) a quantification step in which converting the signal derived from the PSA of interest measured in the measurement step into a quantitative result to give amount of the PSA of interest based on the relationship between the signal derived from the PSA of interest and the amount of the PSA of interest,
wherein, in the quantification step, at least one PSA with a sugar chain represented by any of formulae A to D is used as a reference sample, concentrations of the reference sample in the standard substance are adjusted to predetermined concentrations, the signal from the reference sample is obtained by using the same method as in the measurement step, and the relationship between the obtained signal and the predetermined concentrations is used as the quantitative information.

It is preferable from the viewpoints of accuracy, simplicity, and the like that the measurement method used in the measurement step is column chromatography, mass spectrometry, or ligand binding assay. It is preferable to use surface plasmon field-enhanced fluorescence spectroscopy for the detection in the ligand binding assay.

A lectin and an antibody whose affinity for PSA having β-N-acetylgalactosamine residue at a terminal of the sugar chain is higher than that for PSA not having β-N-acetylgalactosamine residue at any terminal of the sugar chain are preferably used as capture molecules for higher accuracy.

The lectin and the antibody are preferably labeled with a fluorescence dye. Furthermore, it is preferable that the lectin is *Wisteria floribunda* agglutinin (WFA), soybean agglutinin (SBA), *Vicia villosa* lectin (VVL), or *Trichosanthes japonica* agglutinin (TJA-II). It is preferable from the viewpoints of accuracy to use the lectin and the antibody as secondary capture molecules for the ligand binding assay.

The ligand binding assay is a method for quantifying the concentration of an analyte using a "binding reagent (ligand)" that binds specifically to the analyte.

As the binding reagent, an antibody to the analyte is often used, and many of the ligand binding assay methods are immunological measurement methods using the antigen-antibody binding (immunoassays).

Ligand binding assay methods that are the most often used are EIAs (enzyme immunoassays) that use enzyme-labeled antibodies.

The most common methods of the above-mentioned EIAs are non-competitive ELISAs (enzyme-linked immunosorbent assays). Usually, a sample is added to a plate (solid phase) on which the binding reagent is immobilized, the analyte is bound to the immobilized binding reagent, another binding reagent (enzyme-labeled antibody) is further bound to the analyte, and the analyte bound to the solid phase is detected by means of color development, light emission, or signal obtained by the conversion of fluorescence substrate as an index.

The concentration of the analyte in the sample is calculated with the calibration curve prepared using a standard substance. There are various ligand binding assays such as RIAs (radio immunoassays) that uses radioisotope-labeled antibodies, ECLIAs (electrochemiluminescence immunoassays) that uses Ru (ruthenium)-labeled antibodies, and TRFIAs (time-resolved fluorescence immunoassays) that uses lanthanoid-labeled antibodies in addition EIAs that uses enzyme-labeled antibodies as binding agents.

In many ligand binding assay methods, the binding reagent and the drug are bound on a solid plate, and the optical signal is detected with a plate reader. The ligand binding assay methods used in the present invention are not limited to those using a solid plate, and may include a continuous flow process that uses a device having a flow path such as a SPR (surface plasmon resonance) measuring device.

Analytical instruments such as SPFS (surface plasmon-field enhanced fluorescence spectroscopy) using flow paths and reaction cuvette type measurement chips may be used.

### 3.1 Step of measuring signal derived from PSA to be quantified and quantification step

In the measurement step of measuring the signal derived from the PSA, a conventional well-known measurement method can be used. As the measurement and quantification methods, surface plasmon-field enhanced fluorescence spectroscopy (SPFS) is described as a typical example hereinafter.

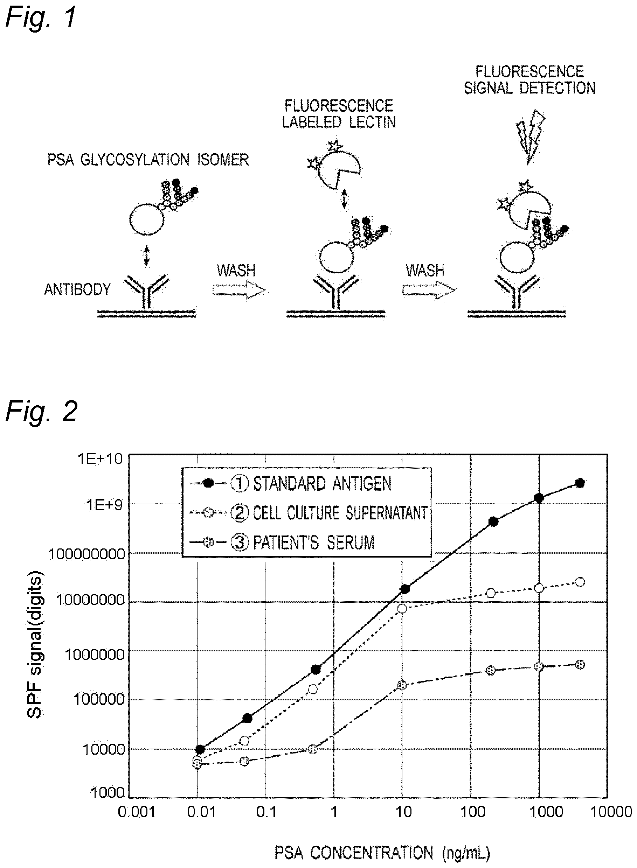

In the measurement and quantification methods using surface plasmon-field enhanced fluorescence spectroscopy (SPFS) in general, a fluorescence signal is measured by sequentially performing the following reaction steps as shown in Figure 1. However, the measurement and quantification methods are not limited to a method according to the following steps.
(i) a step of immobilizing (capturing) an analyte in a sample to an antibody
(ii) a washing step
(iii) a step of labeling a lectin with a fluorescence substance
(iv) a washing step
(v) a step of measuring fluorescence signal intensity

Hereinafter, the measurement and quantification methods, main technical elements related thereto, and the like will be described in detail.

### (a1) Surface plasmon-field enhanced fluorescence spectroscopy (SPFS)

As a method for measuring the signal derived from the PSA, surface plasmon-field enhanced fluorescence spectroscopy (SPFS) is used appropriately.

SPFS is a method for efficiently exciting a fluorescence label on an analyte (object to be analyzed) captured near a metal thin film using a phenomenon in which evanescent waves transmitted through the metal thin film is increased by a factor of several tens to several hundreds by resonance with surface plasmons and measuring fluorescence signals thereof when the metal thin film formed on a dielectric member is irradiated with excitation light at an angle at which attenuated total reflection (ATR) occurs. SPFS has very high sensitivity as compared with conventional methods using fluorescent label. Even when only very a small amount of the analyte is present in a sample, the analyte can therefore be quantified.

The method of measuring a signal derived PSA having a specific residue at a terminal of the sugar chain according to the present invention may preferably be a method for quantifying PSA having, for example, β-N-acetylgalactosamine residue at a terminal of the sugar chain using SPFS. In said method, a lectin whose affinity for PSA having β-N-acetylgalactosamine residue at a terminal of its sugar chain is higher than that for PSA not having the residue at any terminal of its sugar chain is used as a capture molecule.

The lectin may be any that can bind to the residue and examples of the lectins may include *Wisteria floribunda* agglutinin (WFA), soybean agglutinin (SBA), *Vicia villosa* lectin (VVL), and *Trichosanthes japonica* agglutinin (TJA-II). *Wisteria floribunda* agglutinin (WFA) is especially useful. The lectin is preferably labeled with a fluorescent dye.

The quantification method of the present invention is preferably used in a method wherein the above-discussed lectin is used as a secondary capture molecule, such as sandwich assay, competitive assay, and immunoprecipitation assay.

As used herein, "PSA having β-N-acetylgalactosamine residue at a terminal of the sugar chain" is referred to also as "PSA having a specific sugar chain" or merely a "specific analyte". "PSA not having the residue at any terminal of the sugar chain" is referred to also as "PSA not having the specific sugar chain" or merely "unspecific analyte". Furthermore, these PSAs are collectively referred to also as merely "analyte". A "lectin labeled with a fluorescence dye" is described also as merely a "fluorescence-labeled lectin" or a "lectin labeled with fluorescence."

When the quantification method of the present invention is performed in a flow path as sandwich assay using a fluorescence-labeled lectin as the above-mentioned lectin, the amount of fluorescence (fluorescence intensity) by the binding between the analyte and the fluorescence-labeled lectin can be measured without washing this fluorescence-labeled lectin strongly after the reaction of the analyte and the fluorescence-labeled lectin.

Characteristics of the SPFS method including that the enhanced electric field by SPFS for the detection of fluorescence signal reaches only in the range of 200 to 300 nm in height from the interface, i.e. approximately a half of the excitation wavelength, the fluorescence-labeled lectin in charge of the binding reaction can be selectively observed almost without exciting the fluorescence dye of the fluorescence-labeled lectin subjected to Brownian motion realize the above, and is a point advantageous to the detection of the reaction of the sugar chain and the lectin having low binding activity.

It is preferable that a sensor chip comprising a transparent base material, a metal thin film formed on one surface of the transparent base material, a self-assembled monomolecular film [SAM] formed on the surface of the metal thin film which is not in contact with the transparent base material, and a ligand immobilized on the surface of the SAM which is not in contact with the metal thin film (a immobilized primary capture molecule, preferably a solid phase primary antibody) be used at the time of performing sandwich assay using SPFS in the present invention.

The sensor chip may preferably comprise a solid phase layer having three-dimensional structure in addition to the transparent base material, the above-mentioned metal thin film, the above-mentioned SAM, and the above-mentioned ligand. The solid phase layer is formed on the surface of the SAM which is not in contact with the metal thin film surface. The ligand (a primary capture molecule, and preferably a primary antibody) is immobilized in the solid phase layer (preferably in the solid phase layer and on the outer surface thereof).

When the quantification method of the present invention is applied to sandwich assay, it is preferable to use the above discussed sensor chip, and
it is preferable that the sandwich assay comprises the following steps (i) to (iii):
(i) contacting a sample containing an analyte with a sensor chip, and then washing out the components contained in the sample other than the analyte bound to the ligand;
(ii) contacting a lectin labeled with a fluorescence dye with the sensor chip obtained in step (i); and
(iii) according to SPFS, irradiating a laser light to the transparent base material from the surface on which the above-mentioned metal thin film is not formed, measuring the amount of fluorescence emitted from the excited fluorescence dye, and calculating the amount of the specific analyte contained in the sample from the result thereof.

### (Capture molecule)

As used herein, the "capture molecule" means a molecule used to capture the specific analyte in not only sandwich assay (the capture molecule may be a primary capture molecule, or may be a secondary capture molecule, and the capture molecule may be labeled with a fluorescence dye, or may not be labeled with a fluorescence dye) but also all the assays using SPFS.

In the present invention, in sandwich assay using the capture molecule or the primary capture molecule and the secondary capture molecule, or the like, at least one capture molecule is a lectin whose affinity for PSA having β-N-acetylgalactosamine residue at a terminal of the sugar chain is higher than that for PSA not having the residue at any terminal of the sugar chain. The lectin is preferably labeled with a fluorescence dye. Preferably used lectin is *Wisteria floribunda* agglutinin [WFA] and more preferably is *Wisteria floribunda* agglutinin [WFA] labeled with a fluorescent dye. It is further preferable that the dissociation rate constant [kd] of the lectin is 1.0 × 10⁻⁶ to 1.0 × 10⁻⁴ (S⁻¹).

WFA is a lectin extracted and purified from seeds of *Wisteria floribunda,* and exhibits strong affinity for GalNAcβ1→4Gal residue and GalNAcβ1→4GlcNAc (LacdiNAc) residue that has β-N-acetylgalactosamine residue (GalNAcβ1→) at the nonreducing terminal.

### (Fluorescence dye)

In the present invention, the fluorescence dye for labeling the lectin is a general term for substances that emit fluorescence by irradiating predetermined excitation light or by excitation using an electric field effect, and the term "fluorescence" may also include various types of emitted light such as phosphorescence.

As long as the fluorescence dye that may be used in the present invention is not subjected to quenching resulting from the extinction of the metal thin film, the type thereof is not particularly limited, and the fluorescence dye may be any of well-known fluorescence dyes. In view of good detection efficiency, preferable fluorescence dyes may include those can be detected by a fluorometer provided with a filter rather than a monochrometer, and have a large Stokes shift.

Examples of such fluorescence dye as above include fluorescence dyes of fluorescein family (produced by Integrated DNA Technologies), fluorescence dyes of polyhalofluorescein family (produced by Applied Biosystems Japan Ltd.), fluorescence dyes of hexachlorofluorescein family (produced by Applied Biosystems Japan Ltd.), fluorescence dyes of coumarin family (produced by Invitrogen Corporation), fluorescence dyes of rhodamine family (produced by GE Healthcare BioSciences Corporation), fluorescence dyes of cyanine family, fluorescence dyes of indocarbocyanine family, fluorescence dyes of oxazine family, fluorescence dyes of thiazine family, fluorescence dyes of squaraine family, fluorescence dyes of chelated lanthanide family, fluorescence dyes of BODIPY(R) family (produced by Invitrogen Corporation), fluorescence dyes of naphthalenesulfonic acid family, fluorescence dyes of pyrene family, fluorescence dyes of triphenylmethane family, and Alexa Fluor(R) dye series (produced by Invitrogen Corporation). Furthermore, fluorescence dyes described in U.S. Patent Nos. 6,406,297, 6,221,604, 5,994,063, 5,808,044, 5,880,287, 5,556,959, and 5,135,717 can also be used in the present invention.

The absorption wavelength (nm) and emission wavelength (nm) of typical fluorescence dyes included in these families have been known, and for example, are shown in Table 1 of Patent Literature 1 by the present inventors.

The fluorescence dye is not limited to the above-mentioned organic fluorescence dyes. Fluorescence rare earth element complex such as Eu and Tb can also function as a fluorescence dye and can be used for the invention. Rare earth element complexes commonly have characteristics that the wavelength difference between excitation wavelengths (around 310 to 340 nm) and emission wavelengths (approximately 615 nm in the case of Eu complexes and approximately 545 nm in the case of Tb complexes) is great, and the lifetimes of fluorescence are as long as several hundred microseconds or more. Examples of marketed rare earth element complex-based fluorescence dyes include ATBTA-Eu³⁺.

In the present invention, when the below-mentioned fluorescence measurement is performed, it is desirable to use a fluorescence dye having the maximum fluorescence wavelength in a wavelength region with a little absorption of the metal contained in the metal thin film. For example, when gold is used as the metal thin film, it is desirable to use a fluorescence dye having a maximum fluorescence wavelength of 600 nm or more to minimize the influence of the absorption of the gold thin film. In this case, it is therefore particularly desirable to use a fluorescence dye such as Cy5 or Alexa Fluor(R) 647 having the maximum fluorescence wavelength in the nearinfrared region. The use of such a fluorescence dye having the maximum fluorescence wavelength in the nearinfrared region is also preferable because the influence of absorption due to iron derived from blood cell components in blood can be minimized also when blood is used as a sample.

When silver is used as the metal thin film, it is meanwhile desirable to use a fluorescence dye having a maximum fluorescence wavelength of 400 nm or more.

These fluorescence dyes may be used singly or in combinations of two or more.

Examples of a method for producing a lectin labeled with a fluorescence dye include a method in which carboxy groups are first added to a fluorescence dye, the carboxy groups are actively esterified by water-soluble carbodiimide [WSC] (for example, 1-ethyl-3-(3-dimethylaminopropyl)carbodiimide hydrochloride [EDC] or the like) and N-hydroxysuccinimide [NHS], and the actively esterified carboxy groups and amino groups on the lectin are subsequently subjected to dehydration reaction using water-soluble carbodiimide for immobilization; a method in which a lectin and a fluorescence dye having isothiocyanate and an amino group, respectively, are reacted for immobilization; a method in which a lectin and a fluorescence dye having a sulfonyl halide and an amino group, respectively, are reacted for immobilization; a lectin and a fluorescence dye having iodoacetamide and a thiol group, respectively, are reacted for immobilization; and a method in which a biotinylated fluorescence dye and a streptavidinylated lectin (or a streptavidinylated fluorescence dye and a biotinylated lectin) are reacted for immobilization.

### (Samples)

Examples of the samples include blood (serum and plasma), urine, snivel, saliva, feces, and coelomic fluids (cerebrospinal fluid, ascites, and pleural effusion). The sample may be suitably diluted with a desired solvent, a buffer solution, or the like for use. The sample may be used after it is properly diluted with a desired solvent, a buffer solution or the like. Of these samples, blood, serum, plasma, urine, snivel and saliva are preferable.

### (Contact)

A preferred embodiment of the "contact" is an embodiment in which the sensor chip and the sample are brought into contact with each other in such a state that the sample is contained in a transport liquid which circulates in the flow path and only one surface of the SPFS sensor chip, onto said surface the ligand having been immobilized, is immersed in the transport liquid.

As above, an embodiment wherein the flow path is mounted on the metal thin film on the sensor chip, and the sample is into contact with the ligand immobilized on the metal thin film so that the analyte in the sample may be captured by the ligand. An embodiment which does not have a flow path may also be acceptable. Even when the flow path is provided, the transport liquid may not only circulate but also flow reciprocatively or in only one direction in the flow path.

### (Flow path)

As a method for forming a flow path in a sensor chip, it is preferable to crimp a sheet made of polydimethylsiloxane [PDMS] and having a flow path height of 0.5 mm on the surface of the sensor chip on which the metal thin film is formed so that the sheet surrounds a part of the sensor chip in which the metal thin film is formed, and subsequently fix the sheet made of polydimethylsiloxane [PDMS] and the sensor chip with fasteners such as screws, and form a flow path.

As the method for forming a flow path on a sensor chip, a sensor substrate is formed on an integrally molded article of a plastic, or a sensor substrate manufactured separately is fixed on an integrally molded article of a plastic, an SAM (or spacer layer comprising a dielectric), a solid phase layer and a ligand are immobilized on the metal thin film surface, and the sensor board can then be lidded with an integrally molded article of a plastic corresponding to a flow path top plate to also manufacture a flow path on a sensor chip.

### (Liquid feeding)

The temperature of the transport liquid and the circulation time for circulating the transport liquid vary depending upon the type of the sample, etc. and are not specifically restricted, but usually is 20 to 40°C ×1 to 60 minutes, and preferably is 37°C ×5 to 15 minutes.

When the quantification method of the present invention is thus implemented in a flow path, it is preferable that the flow rate of the transport liquid is usually 100 µL/minute or more and 10,000 µL/minute or less. The amount of the sample solution provided in the above-mentioned flow path is preferably 5 µL or more and 1,000 µL or less. When the flow rate of the transport liquid and the amount of the sample solution are in the above-mentioned ranges, respectively, unspecific reaction of the lectin can be reduced, and the specific binding of the lectin with the specific sugar chain on the antigen is secured.

### (Sensor chip)

As mentioned above, the sensor chip is preferably used when the quantification method of the present invention is used for sandwich assay. The sensor chip comprises a transparent base material, a metal thin film, and an SAM, and preferably a solid phase layer and a ligand (the above-mentioned primary antibody is preferable).

### (Transparent base material)

In the present invention, the transparent base material is used as a substrate that supports the structure of the sensor chip. In the present invention, the transparent base material is used as a sensor substrate since the below-mentioned metal thin film is irradiated with light through this transparent base material.

As long as the object of the present invention can be achieved, the transparent base material to be used in the present invention is not particularly limited. For example, the transparent base material may be made of glass, or may be made of a plastic such as polycarbonate [PC] or cycloolefin polymer [COP].

The refractive index [nd] of the transparent support at the d line (588 nm) is preferably 1.40 to 2.20, and the thickness thereof is preferably 0.01 to 10 mm, more preferably 0.5 to 5 mm. The size (length×width) of the transparent support is not specifically restricted.

As a resin constituting the transparent base material, cycloolefin polymer is preferable. For example, ZEONEX E48R (referred to as merely "E48R") produced by Zeon Corporation is further preferable. The refractive index of E48R is 1.51 at a wavelength of 632 nm, and the photoelastic coefficient of E48R is 1.73 × 10⁻¹² Pa⁻¹.

The surface of the transparent support is preferably subjected to cleaning with acid and/or plasma before the metal thin film is formed on the surface. The cleaning treatment with acid is preferably carried out by immersing the transparent support in 0.001 to 1 N hydrochloric acid for 1 to 3 hours. As the cleaning treatment with plasma, there can be mentioned, for example, a method of immersing the transparent support in a plasma dry cleaner ("PDC200" manufactured by Yamato Scientific Co., Ltd.) for 0.1 to 30 minutes.

### (Metal thin film)

### (Metal Thin Film)

In the SPFS sensor chip according to the present invention, a metal thin film is formed on one surface of the transparent support. This metal thin film plays a role in causing surface plasmon excitation due to the irradiation with light from a light source, generating electric field and bringing about light emission from a fluorescence dye.

The metal thin film formed on one surface of the transparent support is preferably made of at least one metal selected from the group consisting of gold, silver, aluminum, copper and platinum, and is more preferably made of gold. These metals may be in the form of their alloys. Such metal species are preferable because they are stable to oxidation and field enhancement by the surface plasmons is promoted.

When a glass support is used as the transparent support, it is preferable to form a thin film of chromium, a nickel-chromium alloy or titanium in advance in order to more firmly bond the metal thin film to the glass.

Examples of methods to form the metal thin film on the transparent support include sputtering method, deposition method (deposition by resistance heating, deposition by electron rays, etc.), electroplating method and electroless plating method. From the viewpoint of easy control of thin film-forming conditions, it is preferable to form a thin film of chromium and/or a metal thin film by sputtering or deposition method.

The thickness of the metal thin film is preferably as follows; gold: 5 to 500 nm, silver: 5 to 500 nm, aluminum: 5 to 500 nm, copper: 5 to 500 nm, platinum: 5 to 500 nm, and their alloys: 5 to 500 nm. The thickness of the thin film of chromium is preferably 1 to 20 nm.

From the viewpoint of field enhancement effect, the thickness of the metal thin film is more preferably as follows; gold: 20 to 70 nm, silver: 20 to 70 nm, aluminum: 10 to 50 nm, copper: 20 to 70 nm, platinum: 20 to 70 nm, and their alloys: 10 to 70 nm. The thickness of the thin film of chromium is more preferably 1 to 3 nm.

When the thickness of the metal thin film is in the above range, surface plasmons are easily generated, so that such a thickness is preferable. The size (length×width) of the metal thin film is not specifically restricted.

### (SAM)

SAM [self-assembled monolayer] is formed on a surface of the metal thin film, said surface not being in contact with the transparent support, as a scaffold for immobilizing the solid phase layer, or for the purpose of preventing quenching of molecular fluorescence due to the metal in the case where the SPFS sensor chip is used for an assay method.

As the monomolecules to constitute the SAM, carboxyalkanethiol of about 4 to 20 carbon atoms (available from, for example, Dojindo Laboratories or Sigma-Aldrich Japan Inc.) is usually used, and 10-carboxy-1-decanethiol is particularly preferably used. The carboxyalkanethiol of 4 to 20 carbon atoms is preferable because SAM formed by the use of it is rarely influenced optically, that is, the SAM has properties of high transparency, low refractive index and small film thickness.

The method for forming such SAM is not specifically restricted, and hitherto publicly known methods are employable. For example, a method of immersing the transparent support having the metal thin film formed on its surface in an ethanol solution containing 10-carboxy-1-decanethiol (manufactured by Dojindo Laboratories) can be mentioned. The thiol group of the 1-carboxy-1-decanethiol is bonded to the metal, immobilized and self-assembled on the surface of the metal thin film to form SAM.

Instead of forming the SAM, a "spacer layer composed of a dielectric" may be formed. As a dielectric used for forming such a "spacer layer comprising a dielectric", various optically transparent inorganic substances and natural or synthetic polymers can also be used. Among those, due to excellent chemical stability, manufacturing stability, and optical transparency, it is preferable that the dielectric contains silicon dioxide [SiO₂], titanium dioxide [TiO₂], or aluminum oxide [Al₂O₃].

The thickness of the spacer layer composed of a dielectric is usually 10 nm to 1 mm, and from the viewpoint of stability of resonance angle, the thickness is preferably not more than 30 nm, more preferably 10 to 20 nm. On the other hand, from the viewpoint of field enhancement, the thickness is preferably 200 nm to 1 mm, and from the viewpoint of stability of field enhancement effect, the thickness is more preferably 400 nm to 1,600 nm.

Examples of methods for forming the spacer layer composed of a dielectric include sputtering method, deposition method by electron rays, thermal deposition method, method by chemical reaction using a material such as polysilazane, and coating method using spin coater.

### (Solid phase layer)

The solid phase layer may be formed on the surface of the above-mentioned SAM which is not in contact with the above-mentioned metal thin film, and has a three-dimensional structure.

This "three-dimensional structure" means a structure of the solid phase layer that enables immobilization of the below-mentioned ligand in the three-dimensional space separated from the surface of the "sensor substrate" without limiting to the two dimensions on the substrate surface (and the vicinity thereof).

Such a solid phase layer preferably contains glucose, carboxymethylated glucose and a polymer constituted of at least one monomer selected from the group consisting of monomers included in vinyl esters, acrylic acid esters, methacrylic acid esters, olefins, styrenes, crotonic acid esters, itaconic acid diesters, maleic acid diesters, fumaric acid diesters, allyl compounds, vinyl ethers and vinyl ketones; it more preferably contains a hydrophilic polymer, such as dextran or a dextran derivative, and a hydrophobic polymer constituted of a hydrophobic monomer selected from vinyl esters, acrylic acid esters, methacrylic acid esters, olefins, styrenes, crotonic acid esters, itaconic acid diesters, maleic acid diesters, fumaric acid diesters, allyl compounds, vinyl ethers and vinyl ketones; and dextran such as carboxymethyl dextran [CMD] is particularly preferable from the viewpoints of biocompatibility, inhibition of non-specific adsorption reaction and high hydrophilicity.

The molecular weight of CMD is preferably not less than 1 kDa but not more than 5,000 kDa, more preferably not less than 4 kDa but not more than 1.000 kDa.

The solid phase layer (composed of, for example, dextran or a dextran derivative) preferably has a density of less than 2 ng/mm2. The density of the solid phase layer can be properly controlled according to the type of the polymer used. It is preferable that the polymer is immobilized on the SAM described above in such density range, because an assay signal is stabilized and increased in the case where the sensor chip is used for an assay method.

The mean film thickness of the solid phase layer is preferably not less than 3 nm but not more than 80 nm. This film thickness can be measured by an atomic force microscope [AFM] or the like. It is preferable that the mean of the film thickness of the solid phase layer is in such a range, because an assay signal is stabilized and increased in the case where the sensor chip is used for an assay method.

A method for immobilizing carboxymethyl dextran [CMD] on the surface of SAM, when it is used as the polymer contained in the solid phase layer will be described specifically.

That is, a substrate in which the transparent base material, the metal thin film, and the SAM are layered sequentially is immersed in an MES buffered saline solution [MES] containing 0.01 mg/mL or more and 100 mg/mL or less of the above mentioned carboxymethyl dextran having preferably a molecular weight of 1 kDa or more and 5,000 kDa or less, 0.01 mM or more and 300 mM or less of N-hydroxysuccinimide [NHS], 0.01 mM or more and 500 mM or less of water-soluble carbodiimide [WSC] for 0.2 hour or more and 3.0 hours or less to immobilize carboxymethyl dextran on the SAM.

The density of the resulting solid phase layer can be controlled by the number of reaction sites (number of functional groups of SAM), the ionic strength and pH of the reaction solution, and the WSC concentration to the number of carboxyl groups of the carboxymethyl dextran molecules. The mean film thickness of the solid phase layer can be controlled by the molecular weight of carboxymethyl dextran and the reaction time.

### (Ligand)

In the present invention, a ligand (primary capture molecule) is used for immobilizing (capturing) the analyte in the sample when the sensor chip is used for sandwich assay. Although the ligand may be immobilized on the above-mentioned metal thin film or SAM, it is preferable that the ligand is immobilized inside and outer surface of the above-mentioned solid phase layer, namely the ligand is dispersed and immobilized in the three-dimensional structure of the solid phase layer.

As used herein, the "ligand" refers to a molecule or a molecule fragment that can specifically recognize (or be recognized by) the analyte contained in the sample and can be bound to the analyte. Examples of the "molecule" or "molecule fragment" may include, but not limited to nucleic acids (single-stranded or doublestranded DNAs, RNAs, polynucleotides, oligonucleotides, PNAs (peptide nucleic acids), or nucleosides, nucleotides, and modified molecules thereof), proteins (polypeptides and oligopeptides), amino acids (including modified amino acids), saccharides (oligosaccharides, polysaccharides, and sugar chains), and lipids, or modified molecules and complexes thereof.

Examples of the "protein" may include antibodies. Specific examples may include an anti-α-fetoprotein [AFP] monoclonal antibody (available from Nihon Rinsho, Inc.), an anti-carcinoembryonic antigen [CEA] monoclonal antibody, an anti-CA19-9 monoclonal antibody, and an anti-PSA monoclonal antibody.

As used herein, the term "antibodies" includes polyclonal or monoclonal antibodies, and antibodies and antibody fragments that are obtained by gene recombination.

Examples of the method for immobilizing this ligand include a method in which carboxy groups on a polymer such as carboxymethyl dextran [CMD] having reactive functional groups are actively esterified with a water-soluble carbodiimide [WSC] (for example, 1-ethyl-3-(3-dimethylaminopropyl) carbodiimide hydrochloride [EDC]) and N-hydroxysuccinimide [NHS], and the thus actively esterified carboxy groups and amino groups on the ligand are subjected to dehydration reaction using a water-soluble carbodiimide for immobilization and a method in which carboxy groups that the above-mentioned SAM has and amino groups that a ligand has are subjected to dehydration reaction as mentioned above for immobilization.

It is preferable to immobilize the above-mentioned ligand and then treat the surface of the sensor chip with a blocking agent such as bovine serum albumin [BSA] to prevent non-specific absorption of the analyte and the like to the sensor chip.

The density of the ligand immobilized in the above-mentioned solid phase layer is preferably 1 fmol/cm² or more and 1 nmol/cm² or less, and more preferably 10 fmol/cm² or more and 100 pmol/cm² or less. It is preferable that the density of the ligand is in the above-mentioned range, because the signal strength increases.

### <Device for SPFS>

Preferably, a device that can be loaded with the sensor chip on the surface of which the primary antibody is immobilized, and comprises the structure that enables PSAs bind to the primary antibody, and the lectin labeled with the fluorescence dye binds to the PSA having a β-N-acetylgalactosamine residue at a terminal of the sugar chain among the PSAs, is used as a device for SPFS.

The device comprises, for example, the light source of a laser light, various optical filters, a prism, a cutting filter, a condensing lens and a surface plasmon-field enhanced fluorescence [SPFS] detecting element in addition to the structure that can be loaded with the above-mentioned sensor chip. The device preferably has a liquid-feeding system combined with the above-mentioned sensor chip for handling the sample liquid, washing liquid, or labeled antibody liquid. The liquid-feeding system may be, for example, a microflow path device connected with a liquid-feeding pump. An image sensor such as a CCD image sensor or a photomultiplier is preferably used for the detection.

The device may also comprise a surface plasmon resonance [SPR] detecting element, namely a photodiode as a light-receiving sensor exclusively for SPR; an angle variable part for adjusting the optimal angle for SPR and SPFS (the angle variable part enables angle change from 45 to 85° by synchronizing the photodiode with the light source for finding attenuated total reflection [ATR] conditions with a servo motor. The resolving power is preferably 0.01° or more.); and a computer for processing inputted information in the SPFS detecting element.

In addition to the above, various known light sources, optical filters, cutting filters, condensing lenses, and SPFS detecting elements may be employed in the present invention.

Examples of the pumps for feeding liquid may include a micropump, which is suitable for feeding a very small amount of liquid; a syringe pump, which cannot be applied to circulatory liquid-feeding, but has high feed accuracy and little pulsation; and a tube pump, which may be unsuitable for feeding a very small amount of liquid, but is simple and excellent in handleability. The liquid-feeding means is not limited to the above-mentioned pumps, and various means can be suitably selected and used depending on the object and the use.

Examples of the measuring device for SPFS will be described in more detail.

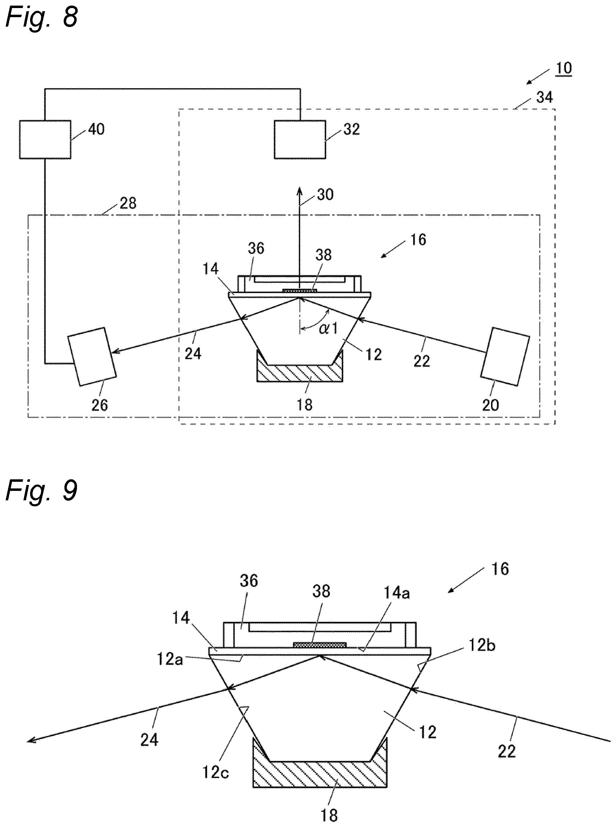

Figure 8 is a schematic diagram showing the outline of a quantitative measuring device for describing the quantitative measurement method for an analyte of the present invention. Figure 9 is a partially enlarged view of the quantitative measuring device of Figure 8.

As shown in Figure 8 and Figure 9, a quantitative measuring device 10 of the present invention comprises a sensor chip 16 having a dielectric member 12 having a prism shape, the vertical cross section shape of which is almost a trapezoid, and a metal film 14 formed on the horizontal upper surface 12a of this dielectric member 12. This sensor chip 16 is loaded into the sensor chip loading part 18 of the quantitative measuring device 10.

As shown in the figure, a light source 20 is disposed in the direction of one lower side 12b of the dielectric member 12 so that incident light 22 from this light source 20 comes in the side 12b of the dielectric member 12 from outside and below the dielectric member 12, and is irradiated toward the metal film 14 formed on the upper surface 12a of the dielectric member 12 through the dielectric member 12.

A polarizing filter for converting the laser light irradiated from the light source 20 into P polarized light, which generates a surface plasmon efficiently on the metal film 14, can also be provided between the light source 20 and the dielectric member 12.

As shown in Figure 8, light-receiving means 26 for receiving metal film-reflected light 24 obtained by reflecting the incidence light 22 on the metal film 14 is provided in the direction of the other lower side 12c of the dielectric member 12.

The light source 20 is provided with incidence angle adjustment means (not shown) that enables suitably changing an incidence angle α1 of the incident light 22 irradiated from the light source 20 to the metal film 14. Meanwhile, the light-receiving means 26 is also provided with unillustrated movable means and configured to certainly receive the metal membrane-reflected light 24 in synchronization with the light source 20 even when the reflection angle of the metal film-reflected light 24 changes.

An SPR measurement part 28 of the quantitative measuring device 10 of the present invention that performs SPR measurement comprises the sensor chip 16, the light source 20, and the light-receiving means 26.

A photo-detection means 32 for receiving fluorescence 30 emitted by exciting a fluorescence substance as mentioned below is provided above the sensor chip 16.

For example, a cut filter, a condensing lens, and the like can also be provided between the sensor chip 16 and the photo-detection means 32.

An SPFS measurement part 34 of the quantitative measuring device 10 of the present invention for performing SPFS measurement comprises the sensor chip 16, the light source 20, and the photo-detection means 32.

The light-receiving means 26 and the photo-detection means 32 are separately connected to a quantitative arithmetic means 40, the light-receiving means 26, the photo-detection means 32, and the quantitative arithmetic means 40 are configured so that the light amount of the metal film-reflected light 24 received by the light-receiving means 26 and the light amount of the fluorescence 30 received by the photo-detection means 32 are transmitted to the quantitative arithmetic means 40.

In the sensor chip 16 of the present Example, a fine flow path 36 is formed on the upper surface 14a of the metal film 14. A sensor part 38 in which a molecule (ligand) that can bind specifically to the antigen to be detected (analyte) is immobilized is provided in a part of this fine flow path 36.

3.2 Others; Devices to be used in a step of measuring signal derived from PSA and quantification step

Following devices are preferably used in combination in the measurement step and the quantification step according to the present invention.

### <Column chromatography>

The analysis (measurement) method using column chromatography comprises: (a) purifying PSA from a sample derived from a subject, (b) preparing a PSA derivative from the PSA purified in step (a), (c) labeling the PSA derivative obtained in step (b), and (d) separating the labeled PSA derivative obtained in step (c) using chromatography such as high performance liquid chromatography or capillary electrophoresis based on the presence of β-N-acetylgalactosamine residue and detecting the signal from the derivative to analyze the sugar chain structure of the PSA.

Step (a) may include, for example, a step of removing albumin in a serum using protein A agarose (PIERCE) or the like, and a step of purifying PSA protein by using anti-PSA antibody beads.

Step (b) may include, for example, a step of cleaving the sugar chain from the PSA protein collected in step (a) using proteinase K, thermolysin, or the like.

Step (c) may include, for example, a step of labeling the PSA-derived sugar chain prepared in step (b) with a compound that can bind to an aldehyde group in a sugar chain such as BOA (benzylhydroxylamine), PA (2-aminopyridine), 2-AA (2-aminobenzamide), 2-AB (2-aminobenzoic acid), or RapiFluor to give the derivative.

A measurement method with a high performance liquid chromatography device equipped with a lectin affinity column (also referred to as a "lectin column") is also preferable.

### <Mass spectrometry>

Various methods can be used as mass spectrometry. For example, liquid chromatography-mass spectrometry (abbreviation: "LC/MS"), matrix-assisted laser desorption/ionization (abbreviation: MALDI), time-of-flight mass spectrometry (abbreviation: TOFMS) and gas chromatography-mass spectrometry (abbreviation: "GC/MS") can be used.

Mass spectrometry can be performed by the following procedures. A sample to be measured is ionized. Various generated ions are then fed to a mass spectroscope, and the ionic strength are measured at each ratio of mass to charge m/z, which is the ratio of the mass number of each ion m to the valence z. The consequently obtained mass spectrum comprises the peaks of the measured ionic strengths versus the values of the ratios of mass to charge m/z (ion peaks).

The mass spectrometry of the thus ionized sample itself is called MS1. In a tandem type mass spectroscope that enables multistage dissociation, an ion peak having the value of a certain specific ratio of mass to charge m/z is selected among the ion peaks detected in MS1 (the selected ionic species is called parent ions), the ions are further dissociated and decomposed by the collision with gas molecules or the like, the generated dissociative ionic species are subjected to mass spectrometry, and a mass spectrum is obtained in the same way. Here, it is called MSn+1 to dissociate the parent ions at n stages and subject the dissociative ionic species to mass spectrometry. In the tandem type mass spectroscope, the parent ions are thus subjected to multistage (first stage, second stage, ...,nth stage) dissociation to analyze the mass numbers of the ionic species generated at the stages (MS2, MS3, ..., MSn+1).

### <Electrophoresis>

Examples of the analysis method using electrophoresis include SDS gel electrophoresis (one dimension), isoelectric focusing (two dimensions), and lectin affinity electrophoresis.

In one-dimensional electrophoresis, as proteins become smaller, the proteins move more rapidly without being captured in meshes of the gel at the time of electrophoresing proteins in a gel, and the proteins can therefore be separated in order of molecular weight. The speed at which the proteins pass through gel meshes is a method for separating and analyzing substances depending on differences in not only the molecular weight of each protein but also the higher order structure, the charge thereof, and the like.

Two-dimensional electrophoresis is a separation method in which the proteins are electrophoresed twice, namely in the X direction and the Y direction. Different separation conditions are combined for the first electrophoresis and the second electrophoresis to make the two-dimensional electrophoresis better in separation ability than one-dimensional electrophoresis. In general, analytes are separated depending on the isoelectric points (subjected to IEF/isoelectric focusing) in the X direction and separated depending on the molecular weights in the Y direction.

The lectin affinity electrophoresis is a method for separating glycoproteins having reactivity with lectin depending on the difference in affinities using the lectin as an affinity medium as a relative immobilized phase.

### Examples

The present invention will be described in more detail by showing Examples and Comparative Examples below. They do not limit the scope of the present invention.

[Example 1]: Preparation of calibration curves with various solutions

The following three solutions were provided for preparing calibration curves.
(a) PSA-glycosylation isomer (hereinafter called "PSA-Gi") standard solution
(b) Culture supernatant
(c) Patient serum's having a high PSA value
Hereinafter, each of the solutions will be described.

### (1.1) Preparation of PSA-Gi standard solution

As a raw material of the standard solution, PSA derived from seminal plasma (having a purity of 96% or more according to SDS-PAGE; produced by Lee BioSolutions, Inc.) was used.

Terminal sialic acid was hydrolyzed using a glycohydrolase, neuraminidase (produced by NACALAI TESQUE, INC.) to modify the sugar chain of PSA derived from seminal plasma into a specific sugar chain structure found in PSAs secreted from cancer cells. Neuraminidase was mixed with the protein at an amount ratio of 0.5 U of neuraminidase to 1 mg of protein, the mixture was left to stand at 37°C for 1.5 hours for the enzyme reaction.

Only PSAs with a sugar chain having an affinity for WFA lectin were purified from the hydrolysis-treated PSAs derived from seminal plasma by lectin affinity column chromatography.

Thus purified PSAs were analyzed using mass spectrometry to confirm if PSAs having a sugar chain other than LacdiNAc-including sugar chain were contained. PSAs that were confirmed to have a sugar chain other than the sugar chain structures of interest were subjected to lectin affinity column chromatography purification repeatedly. The same operation as this was performed a plurality of times for raw materials derived from a plurality of donors.

The isolated and purified PSAs were analyzed for their sugar chains using LC-MS and the contents of the respective sugar chains of interest were calculated. The PSAs derived from donors were mixed to prepare the PSA-Gi standard solution so that the standard solution contains the sugar chains of interest in the desired ratio.

### <Conditions for affinity column chromatography>

In Example 1, a WFA column in which WFA was used as a lectin was produced, and PSAs having the residue of specific structure at a terminal of the sugar chain were isolated and purified.

### (Production of WFA column)

First, 1.5 mL of WFA agarose gel (produced by Vector Laboratories Inc., WFA concentration: 3 mg/mL) was added to Pierce(R) Disposable Columns (produced by Thermo Fisher Scientific) to produce a WFA column filled with a solution of agarose to which WFA was bound at a density of 3 mg per mL.

### (Isolation and purification of PSAs with a sugar chain having the specific structure)

First, 2 mL of a WFA agarose solution was equilibrated with a phosphate buffer solution under the condition of 4°C. Next, 50 µL of the PSA sample after the enzyme treatment was diluted to 200 µL with the phosphate buffer solution, and the diluted sample was applied to the column and retained for 30 minutes.

Then, the column was washed with five times volume of the buffer solution for washing (phosphate buffer solution) and the buffer solution was fractionated into 1-mL fractions to obtain WFA-unbound fractions. The column was returned to room temperature, and the PSA was eluted with five times volume of the elution buffer (0.4 M lactose phosphate buffer solution) and the eluted solution was fractionated into 1-ml fractions to obtain WFA-bound fractions. The elution pattern of WFA fractions was measured for the PSA-Gi concentrations by SPFS measurement, and WFA-bound fractions were obtained.

### <LC-MS measurement conditions>

The WFA-bound fractions were subjected to solvent exchange to exchange the 0.4 M lactose phosphate buffer solution by ultrafiltration to give a sample for LC-MS measurement. After the solvent exchange, the absorbances were measured and the PSA concentrations were calculated. N-glycan chains on the PSAs were cleaved and subjected to RapiFluor labeling reaction using a GlycoWorks RapiFluor-MS N-Glycan Kit (produced by Waters Corporation). The prepared sugar chain samples were analyzed under the following conditions.

### (LC conditions)

Column: ACQUITY UPLC Glycan BEH Amide, 130 A, 1.7 µm, 2.1 mm × 150 mm
Mobile phase A: 50 mM Ammonium formate solution, pH 4.4 Mobile phase B: Acetonitrile
Concentration gradient: A/B = 25/75 (0 min) - 46/54 (35.0 min)
Analysis time: 55 min
Column temperature: 60°C
Flow rate: 0.4 mL/min
Injection amount: 10 µL

### (MS conditions)

Ionization and polarity: ES+
Analyzer: Sensitivity Mode
Capillary voltage (kV): 3.0
Sampling cone: 80.0
Source temperature (°C):120
Source offset: 80
Desolvation temperature (°C): 300
Desolvation gas flow rate Flow (L/Hr): 800

In the analysis, the extra ion chromatogram of m/z corresponding to the N-glycan chains of interest was extracted, and the peak areas were calculated using MassLynx software. The contents of the sugar chain structures were calculated from the ionic strengths of the sugar chains of interest.

### (1.2) Preparation of culture supernatant

The medium of a semiconfluent culture of prostatic cancer cell line LNCaP (ATCC CRL-1740) in RPMI-1640 serum-free medium (produced by Thermo Fisher Scientific K.K.) was exchanged and cultured two more days, and then, the culture supernatant was collected.

### (1.3) Preparation of patient's serum having high PSA value

Patient's serum having a PSA concentration determined by ELISA as high as 1000 ng/mL or more was used as the patient's serum having a high PSA value,

### (1.4) Acquisition of data for calibration curve by SPFS measurement

The PSA concentrations in the various solutions obtained by the above-mentioned preparation methods were measured, and series of diluted solutions at PSA concentrations were prepared with a 1% BSA/PBS solution. The fluorescence signal was measured basically along with the reaction steps shown in Figure 1 with the SPFS measuring device using the prepared solutions as samples.

### <Measurement by surface plasmon field-enhanced fluorescence spectroscopy>

Measurement by surface plasmon field-enhanced fluorescence spectroscopy ("SPFS") was performed as follows.

### (Manufacturing of plasmon sensor)

A 1-mm-thick glass transparent flat substrate "S-LAL10" (manufactured by OHARA INC., refractive index [nd] = 1.72) was subjected to plasma cleaning with the plasma dry cleaner "PDC200" (produced by Yamato Scientific Co., Ltd.). A chromium film was first formed on one surface of the substrate subjected to plasma cleaning by sputtering, and a gold film was further formed on its surface by sputtering. The thickness of this chromium film was 1 to 3 nm, and the thickness of the gold film was 44 to 52 nm.

The thus obtained substrate was subsequently immersed in 10 mL of a solution of 10-carboxy-1-decanethiol in ethanol adjusted to 25 mg/mL for 24 hours to form a self-assembled monolayer (SAM) on the surface of the gold film. This substrate was taken out of the ethanol solution, washed with ethanol and isopropanol sequentially, and dried using an air gun.

The substrate on which the SAM was formed on the surface was subsequently immersed in an MES buffer solution containing 1 mg/mL carboxymethyl dextran [CMD] having a molecular weight of 500,000, 0.5 mM N-hydroxysuccinimide [NHS], and 1 mM water-soluble carbodiimide [WSC] for 1 hour. The CMD was immobilized on the SAM, and then, the unreacted succinate ester was hydrolyzed by immersing the substrate in a 1 N aqueous NaOH solution for 30 minutes.

A sheet-like 0.5-mm-thick silicone rubber spacer having a hole (2 mm × 14 mm) was provided on the obtained substrate on the surface on which the gold film + the SAM + the solid phase layer having three-dimensional structure were formed sequentially. The substrate was disposed so that the surface was inside a flow path (wherein the silicone rubber spacer does not contact with fed liquid), the 2-mm-thick polymethyl methacrylate plate was placed thereon and crimped so as to cover the substrate from outside the flow path, and the flow path and the polymethyl methacrylate plate were fixed with screws.

Ultrapure water was fed and circulated for 10 minutes and subsequently PBS was fed and circulated for 20 minutes at room temperature and a flow rate of 500 µL/min with the peristaltic pump. Then, 5 mL of a PBS containing 50 mM NHS and 100 mM WSC was fed and circulated for 20 minutes, and 2.5 mL of an anti-PSA monoclonal antibody (produced by Mikuri immunological laboratory corporation) solution was then fed and circulated for 30 minutes to immobilize the primary antibody on the solid phase layer having three-dimensional structure. PBS buffered saline solution containing 1% by mass bovine serum albumin [BSA] was finally fed and circulated for 30 minutes for preventing unspecific adsorption to manufacture a plasmon sensor.

### (Fluorescence-labeling of WFA lectin)

WFA *[Wisteria floribunda* Agglutinin] (Vector) was fluorescence-labeled using an Alexa Fluor (trade name) 647 protein labeling kit (produced by Invitrogen Corporation).

The procedure was performed according to the protocol attached to the kit. To remove the unreacted lectin, the unreacted fluorescence, and the like, the reaction product was purified using an ultrafiltration membrane (manufactured by Nihon Millipore K.K.) to obtain an Alexa Fluor 647-labeled WFA solution. The obtained solution of the fluorescence-labeled lectin was stored at 4°C after protein quantification.

### (Measurement by SPFS)

First washing step: A tris buffered saline solution containing 0.05% by mass Tween(R) 20 was fed and circulated for 10 minutes.

Blank measurement step: The flow path was filled with tris buffered saline. A laser light of 635 nm wavelength was irradiated onto the metal thin film from the back surface of the plasmon excitation sensor through the prism, with the photon amount adjusted by an optical filter. A photomultiplier tube installed at the top of the measurement area measured the intensity of light passing through the filter, which cuts off wavelengths other than the fluorescence component. This measurement is a blank value because it is a noise value that does not include the fluorescence emitted by the WFA-labeled fluorophore.

Primary reaction step: The prepared serum sample was fed and circulated for 30 minutes in the flow path in which the anti-PSA antibody is immobilized was formed.

### Second washing step: A tris buffered saline solution containing 0.05% by mass Tween(R) 20

Secondary reaction step: The prepared labeled WFA lectin solution was fed and circulated for 10 minutes.

Third washing step: A tris buffered saline solution containing 0.05% by mass Tween(R) 20 was fed and circulated for 10 minutes.

Signal measurement step: The flow path was filled with tris buffered saline. A laser light of 635 nm wavelength was irradiated onto the metal thin film from the back surface of the plasmon excitation sensor through the prism, with the photon amount adjusted by an optical filter. A photomultiplier tube installed at the top of the measurement area measured the intensity of light passing through the filter, which cuts off wavelengths other than the fluorescence component. (signal value).

### (1.5) Measurement result

Calibration curve as shown in Figure 2 was obtained based on the data obtained by the above-mentioned measurement. The signal increased in response to the concentrations of PSA.

The results of Figure 2 shows that (a) PSA-Gi standard solution has the widest measurable concentration range. Since the ratio of PSA-Gi to the total PSA in patient's serum itself (c) is low and PSAs having irrelevant sugar chains are competitive to PSA-Gi in the system using anti-PSA antibody as the solid phase antibody, the concentrations of lower detection limit increases and of upper detection limit decreases. This causes the very limited quantifiable concentration range and the sample solution is not suitable for the measurement of patient's samples having wide varieties of PSA concentrations.

In contrast to patient's serum itself (c), the cell culture supernatant (b) could provide a certain measurement range and it was around a 3-digit dynamic range. The culture supernatant (b) can be used as a standard solution in view of the concentration range commonly required in clinical examinations.

Although the sugar chain expression patterns are unbalanced in some cultured cells, it is confirmed that, for example, at least culture supernatants of prostatic cancer cell line LNCaP and VCaP cells exhibit reactivity comparative to the (a) PSA-Gi standard solution.

### [Example 2]: Confirmation of purity by electrophoresis

Electrophoretic analysis by SDS-PAGE was performed for confirming the purity of the prepared PSA-Gi standard substance as protein.

Precision Plus Protein(TM) Prestained Standards (Bio-Rad Laboratories, Inc., #1610373), which is a protein molecular weight marker, was set in lane 1, the prepared PSA-Gi standard substances were set in lanes 2 and 3, and electrophoresis was performed using 10% Mini-PROTEAN(R) TGX(TM) Precast Gels (Bio-Rad Laboratories, Inc., #4561033).

After the electrophoresis, CBB staining was performed using Imperial(TM) Protein Stain (Thermo Fisher Scientific K.K. #24615).

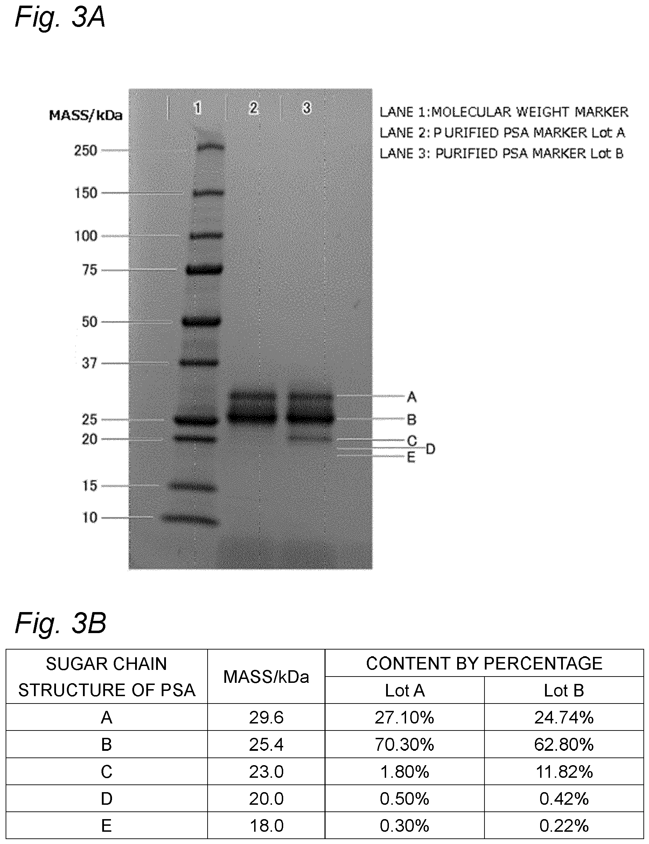

Then, the band staining intensities of the lanes were analyzed with a Gel Doc EZ gel photographing device (Bio Rad Laboratories, Inc.). Figure 3 (A and B) shows the analysis results.

The detected bands were 5 bands in total as a result of the band staining intensity analysis. It is presumed that the bands confirmed in the range of 23 to 33 kDa are the bands of PSA proteins.

The bands at a molecular weight of 23 kDa or less are considered to be decomposed substances of PSA or impurities. It has been confirmed that the band components having 23 to 33 kDa account for 95% or more of the whole.

Apparent protein components were not contained except the PSA protein components of the PSA-Gi standard substance, and it has been confirmed that the PSA protein purity was 95% or more as a result of the densitometry analysis of the detected bands. It was confirmed that the standard substance had PSA components contained in protein with high purity.

### [Example 3]: Profile analysis of the PSA sugar chain structures

The N-type sugar chains with which the prepared PSA-Gi standard substance was modified were analyzed by LC-MS for profile analysis.

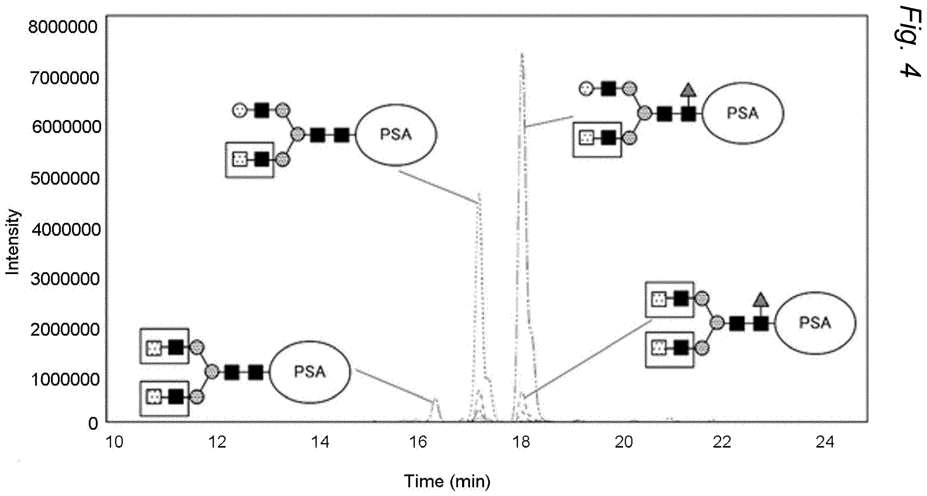

Figure 4 shows an extra ion chromatogram of components contained at 1% or more with respect to the total of all the detected sugar chain peaks.

In addition to the PSAs with any of the four structures having GalNAc residue at a terminal of the sugar chain, components having Gal residues on both of the branched terminals, namely PSAs having a sugar chain represented by formula E or F were confirmed as slight impurities. The amount of those impurities was around 5% of the whole PSAs, and the influence of the impurities on the reactivity is presumed to be kept very small.

The main components are two components that are PSAs having sugar chains of formula A or B. These are components having either of the two branches of the sugar chain has GalNAc, and are frequently detected in samples obtained from cancer patients. The amount of those two components is around 90% of the whole PSA-Gi standard substance.

The standard substance also comprises PSAs having a sugar chain of formula (C) or (D) which comprises GalNAc on both of two branches of the sugar chain in a content of around 5 to 10% in total as minor components. It is assumed that the effect of fucose residue, which is the difference between formulas A and B, and between C and D has little effect on WFA binding, considering the size of the glycan recognition pocket of WFA and other factors.

Figure 4 also shows data of PSA-Gi standard substances which were prepared from multiple different donors. It has been confirmed from the results shown in Figure 4 that the reproducibility of the component contents in the prepared PSA-Gi standard substances can be controlled to some extent.

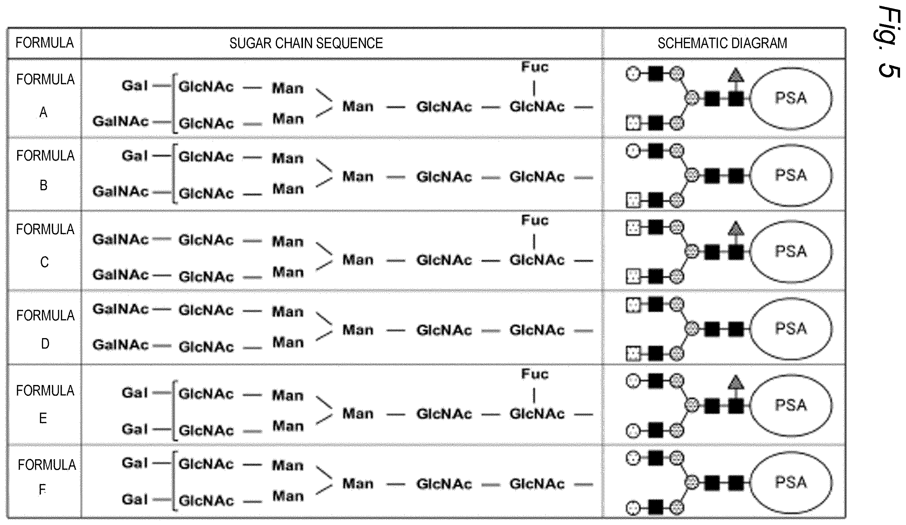

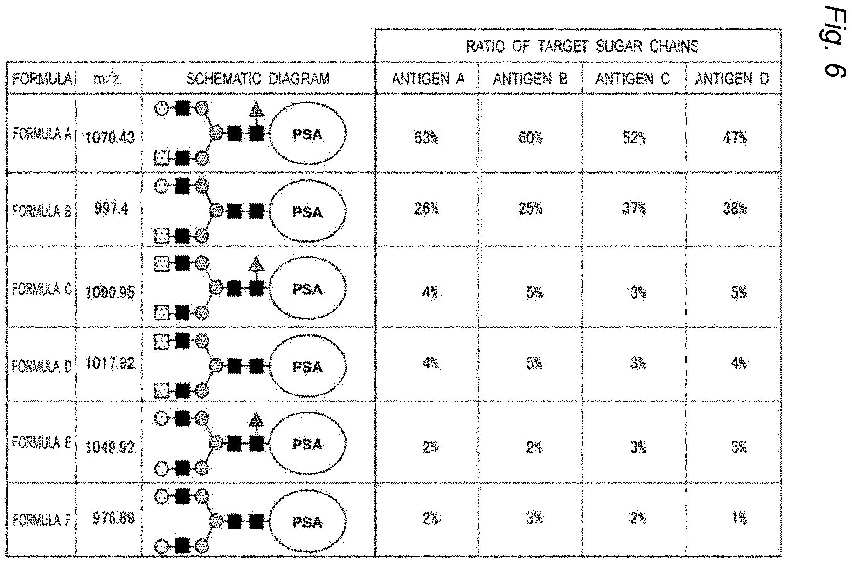

Figures 5 and 6 show information of sugar chain sequences represented by formulae A to F and the ratio of respective sugar chain in the PSA-Gi standard substance obtained from different donors.

### [Example 4]: Test on dilution linearity of samples

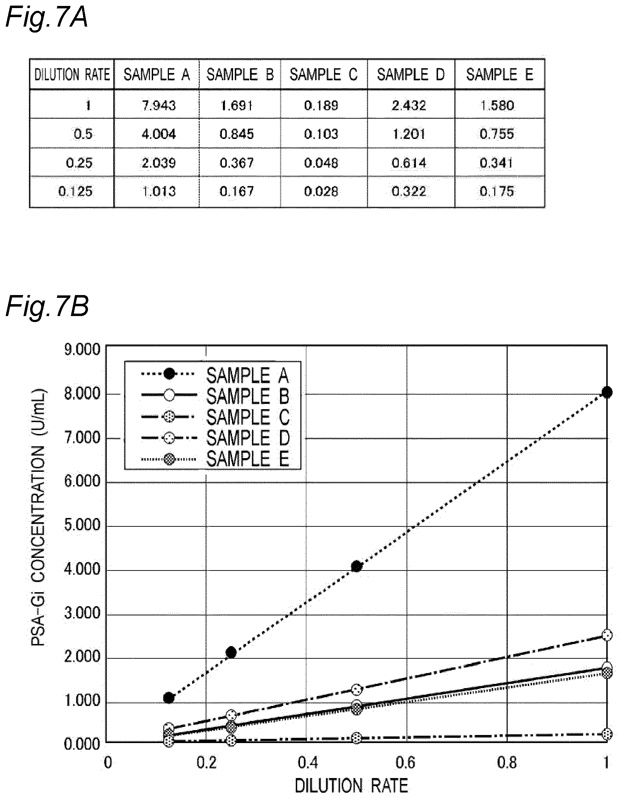

A plurality of patient's samples (serums) were measured using the calibration curve generated with the prepared PSA-Gi standard substance. The patient's serums were serially diluted and measured. The dilution linearity of each sample was determined. Figure 7 shows the evaluation results.

As shown in Figure 7A and Figure 7B, high dilution linearity of the quantified values were obtained in five different patient's samples.

This result shows that the standard substance used for generating the calibration curve and the antigen components contained in the patient's samples exhibit similar reactivity with the measurement reagents. This result indicates that PSAs in the patient's sample have sugar chains homologous to the sugar chain structures of PSAs contained in the standard substance. It has been confirmed that the ratio of the sugar chains is also similar to those contained in the standard substance.

### Industrial Applicability

According to the procedures provided by the present invention, a standard substance for PSA quantification, which has less unbalanced sugar chain expression patterns, which can be manufactured with high reproducibility, and which enables general-purpose quantification including the quantification of patient's sample comprising a high concentration of PSA can be provided. The present invention also provides a preparation method therefor, a standard solution for PSA quantification, and a PSA quantification method.

### Reference Signs List

10: Quantitative measuring device
12: Dielectric member
   12a: Upper surface 12b: Side
   12c: Side
14: Metal film
   14a: Upper surface
16: Sensor chip
18: Sensor chip loading part
20: Light source
22: Incident light
24: Metal film reflected light
26: Light-receiving means
28: SPR measurement part
30: Fluorescence
32: Photo-detection means
34: SPFS measurement part
36: Fine flow path
38: Sensor part
40: Quantitative arithmetic means

## Claims

1. A standard substance for quantification of prostate specific antigen (hereinafter referred to as "PSA") having a specific sugar chain,
wherein the standard substance comprises a compound having the structure of a PSA with a sugar chain represented by any of the following formulae A to D: 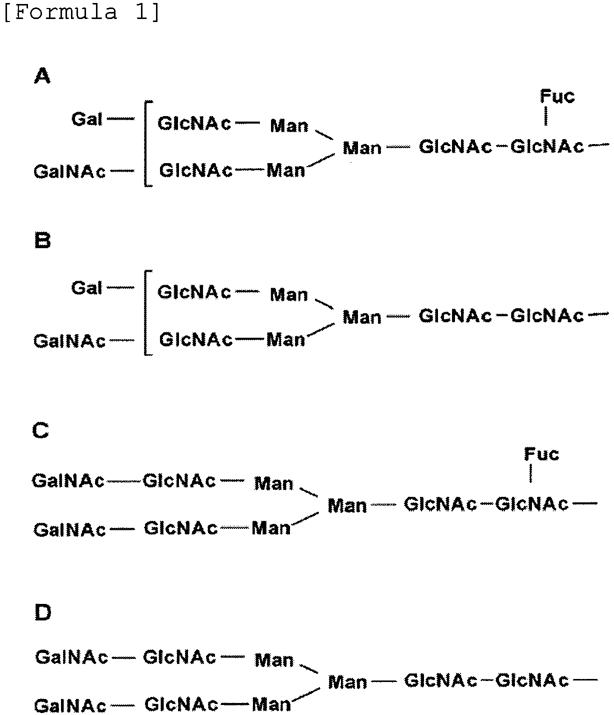 wherein the compound is isolated and purified from a natural product, the compound is chemically or enzymatically altered from a natural product, or the compound is artificially synthesized.

2. A method for preparing the standard substance for PSA quantification of claim 1, which is any method of the following (1) to (4):
(1) a method which comprises isolating, purifying, diluting or concentrating a PSA with a sugar chain of interest from PSAs in a raw material that is derived from a human, cultured cells, or fungi,
(2) a method which comprises chemically or enzymatically decomposing or synthesizing a sugar chain on a PSA in a raw material that is derived from a human, cultured cells, or fungi to form PSA with a sugar chain of interest, and isolating, purifying, diluting, or concentrating the PSA with the sugar chain of interest,
(3) a method which comprises exchanging the sugar chain on a PSA in a raw material that is derived from a human, cultured cells or fungi with an artificially synthesized sugar chain by utilizing a chemical or enzymatic substitution reaction, and
(4) a method which comprises manipulating a gene encoding for an enzyme involved in the sugar chain biosynthesis in cells or fungi by means of gene recombination technology, and using the cells or fungi to produce the compound.

3. A standard solution for PSA quantification, comprising the standard substance for PSA quantification according to claim 1, wherein the standard solution comprises a PSA with a sugar chain represented by any of formulae A to D.

4. The standard solution for PSA quantification according to claim 3, comprising the PSA having the sugar chains of formula A or B, wherein the total amount of both the PSAs having the sugar chain of formula A or B is 80% by mass or more of the total amount of the PSAs having the sugar chain of any one of formulae A to D.

5. The standard solution for PSA quantification according to claim 3, comprising 30 to 70% by mass of PSA with the sugar chain of formula A, 20 to 50% by mass of PSA with the sugar chain of formula B, 1 to 10% by mass of PSA with the sugar chain of formula C, and 1 to 10% by mass of PSA with the sugar chain of formula D on the basis of the total amount of PSAs having a sugar chain of any of formulae A to D.

6. A method for quantifying PSA using the standard substance for PSA quantification according to claim 1 or the standard solution for PSA quantification according to any one of claim 3 to claim 5, comprising:
a measurement step in which a signal derived from PSA to be quantified in a sample is measured; and
a quantification step in which converting the signal derived from the PSA of interest measured in the measurement step into a quantitative result to give amount of the PSA of interest based on the relationship between the signal derived from the PSA of interest and the amount of the PSA of interest,
wherein, in the quantification step, at least one PSA with a sugar chain represented by any of formulae A to D is used as a reference sample, concentrations of the reference sample in the standard substance are adjusted to predetermined concentrations, the signal from the reference sample is obtained by using the same method as in the measurement step, and the relationship between the obtained signal and the predetermined concentrations is used as the quantitative information.

7. The method for quantifying PSA according to claim 6, wherein the measurement procedure used in the measurement step is column chromatography, mass spectrometry, or ligand binding assay.

8. The method for quantifying PSA according to claim 7, wherein surface plasmon field-enhanced fluorescence spectroscopy is used for the detection in the ligand binding assay.

9. The method for quantifying PSA according to any one of claim 6 to claim 8, wherein a lectin and an antibody whose affinities for PSA having β-N-acetylgalactosamine residue at a terminal of the sugar chain are higher than that for PSA not having β-N-acetylgalactosamine residue at any terminal of the sugar chain are used as capture molecules.

10. The method for quantifying PSA according to claim 9, wherein the lectin and the antibody are labeled with a fluorescence dye.

11. The method for quantifying PSA according to claim 9 or claim 10, wherein the lectin is *Wisteria floribunda* agglutinin (WFA), soybean agglutinin (SBA), *Vicia villosa* lectin (VVL), or *Trichosanthes japonica* agglutinin (TJA-II) .

12. The method for quantifying PSA according to any one of claim 7 to claim 11, wherein the lectin and the antibody are used as secondary capture molecules for the ligand binding assay.
